# EUROPÄISCHE PATENTSCHRIFT

(11) **EP 0 617 019 B1**
(45) Veröffentlichungstag und Bekanntmachung des Hinweises auf die Patenterteilung: **31.05.2000**
(21) Anmeldenummer: 94103776.4
(22) Anmeldetag: 11.03.1994
(51) Int. Cl.: C07D 213/55, C07D 401/06

(54) **Verfahren zur Herstellung von 3R,5S-(+)-Natrium-erythro-(E)-7-[4-(4-fluorphenyl)--2,6-diisopropyl-5-methoxymethyl-pyrid-3-yl]-3,5-dihydroxy-hept-6-enoat**
Process for the preparation of 3R,5S-(+)-sodium-erythro-(E)-7-4-(4-fluorophenyl)--2,6-diisopropyl-5-methoxymethyl-pyrid-3-yl-3,5-dihydroxy-hept-6-enoate
Procédé de préparation de 3R,5S-(+)-érythro-(E)-7-4-(4-fluorophényl)--2,6-diisopropyl-5-méthoxyméthyl-pyrid-3-yl-3,5 dihydroxy-hept-6-énoate de sodium

(30) Priorität: 24.03.1993 DE 4309553
(43) Veröffentlichungstag der Anmeldung: 28.09.1994
(73) Patentinhaber: BAYER AG, 51368 Leverkusen (DE)
(72) Erfinder: Angerbauer, Rolf, Dr., D-42113 Wuppertal (DE); Grosser, Rolf, Dr., D-51373 Leverkusen (DE); Hinsken, Werner, Dr., Miles Incorporated, Clayton, NC 27520 (US); Rehse, Joachim, Dr., D-42105 Wuppertal (DE)

(56) Entgegenhaltungen:
- EP-A- 0 306 929
- EP-A- 0 325 130
- EP-A- 0 491 226

## Beschreibung

Die vorliegende Erfindung betrifft ein Verfahren zur Herstellung von enantiomerenreinem 3R,5S-(+)-Natrium-erythro-(E)-7-[4-(4-fluorphenyl)-2,6-diisopropyl-5-methoxymethyl-pyrid-3-yl]-3,5-dihydroxy-hept-6-enoat.

In der europäischen Anmeldung EP 491 226 wird die Verbindung der Formel (I) 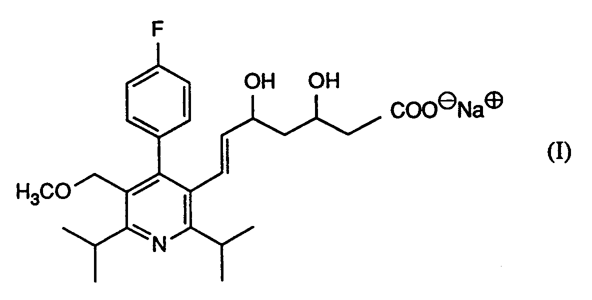 beschrieben, die aufgrund ihrer Eigenschaft, die Cholesterinbiosynthese zu hemmen, in Arzneimitteln zur Behandlung von Lipoproteinämie eingesetzt werden kann. In dieser europäischen Patentanmeldung wird diese Substanz in Form ihres Racemates sowie der beiden Enantiomeren offenbart. Die Herstellung des Racemates erfolgt durch die Hydrolyse des entsprechenden racemischen Esters, der bereits in der europäischen Patentanmeldung EP 325 130 beschrieben ist. Die Herstellung der beiden Enantiomeren erfolgt durch Umsetzen des racemischen Esters mit dem optisch aktiven R-(+)-Phenylethylamin zu dem Diastereomerengemisch der entsprechenden Amide, die wiederum nach üblichen Verfahren der Chromatographie in die einzelnen Diastereomeren getrennt werden. Durch Abspaltung der Amidgruppe aus den getrennten Diastereomeren erhält man das jeweils gewünschte enantiomerenreine Produkt. Diese Vorgehensweise sei im folgenden Schema kurz dargestellt.

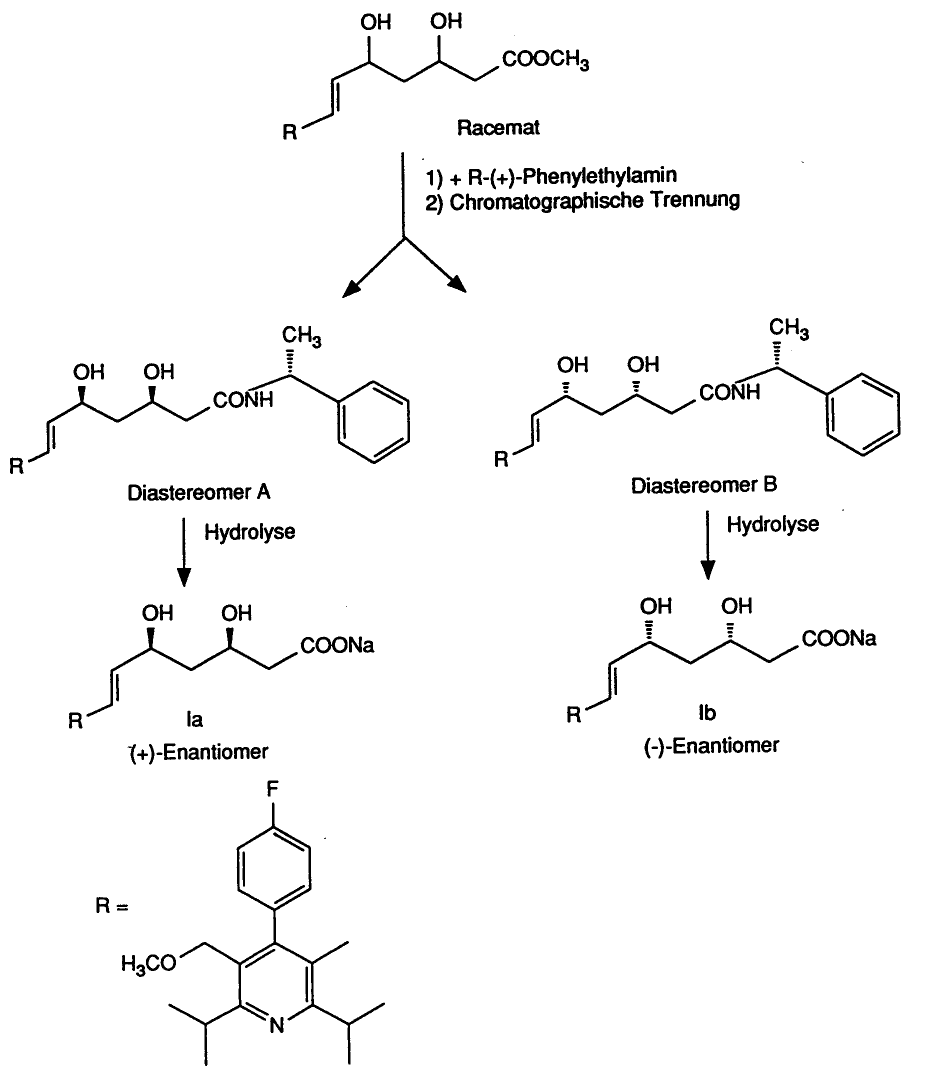

Ein wesentlicher Nachteil dieses Verfahrens ist, daß die Separierung des Racemates nicht durch direkte Chromatographie erfolgen kann, sondern zunächst eine Überführung in die diastereomeren Amide erfolgen muß, die nach der chromatographischen Trennung durch eine erneute chemische Behandlung (Hydrolyse) in die reinen Enantiomeren übergeführt werden können. Dies bedeutet einerseits einen hohen technischen Aufwand und andererseits einen Ausbeuteverlust allein durch die Tatsache, daß bei zwei chemischen Umsetzungen und einer chromatographischen Trennung Produkt verlorengeht.

EP-A-0306929 offenbart ein Verfahren zur Herstellung von 3R,5S-(+)-Natrium-erythro-(E)-[4-(fluorphenyl)-2,6-diisopropyl-5-methoxymethyl-pyrid-3-yl]-3,5-dihydroxy-hep-6-enoat. Die Trennung der Enantiomere erfolgt auf der Stufe der racemischen Dihydroxyester. Eine Trennung an chiralen Phasen ist nicht offenbart.

Es wurde nun gefunden, daß das 3R,5S-(+)-Natrium-erythro-(E)-7-[4-(4-fluorphenyl)-2,6-diisopropyl-5-methoxymethyl-pyrid-3-yl]-3,5-dihydroxy-hept-6-enoat der Formel (Ia) 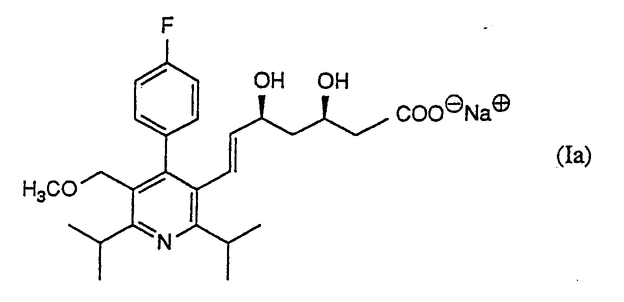 in enantiomerenreiner Form hergestellt werden kann, indem man zunächst racemische Ester der Formel (II) 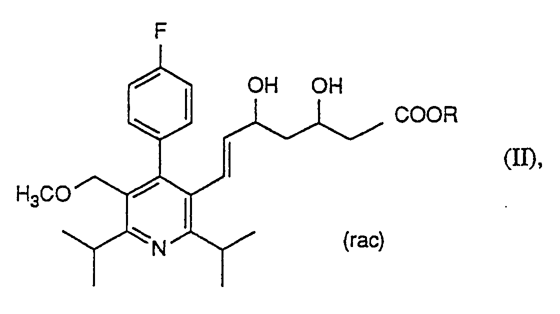 in welcher
- R: für C₁-C₄-Alkyl steht,
in die racemischen Lactone der Formel (III) überführt, 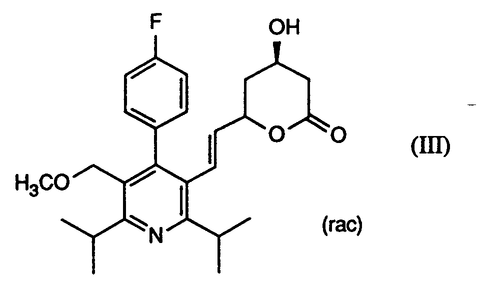 dieses Racemat durch Chromatographie an einer chiralen Phase in die einzelnen enantiomeren Lactone separiert und die enantiomeren Lactone jeweils durch Hydrolyse in das gewünschte enantiomerenreine Endprodukt überführt.

Das erfindungsgemaße Verfahren kann durch folgendes Schema erläutert werden:

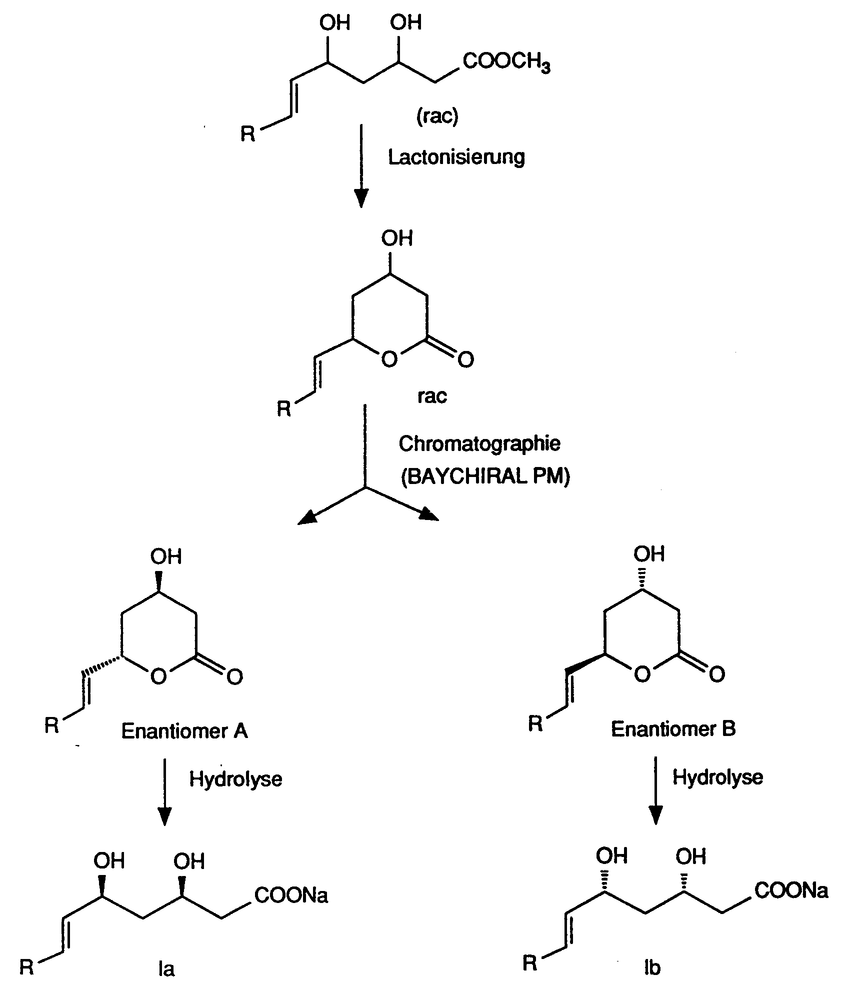

Die Überführung der racemischen Carbonsäureester (III) in die racemischen Lactone (IV) erfolgt im allgemeinen durch Behandeln des Ester mit Basen und anschließende Cyclisierung in geeigneten Lösemitteln unter Wasserabspaltung. Bei dieser Reaktion entstehen intermediär die Carbonsäuren bzw. deren Salze.

Als Basen hierfür eignen sich die üblichen anorganischen Basen wie Natriumhydroxid, Kaliumhydroxid, Natriumcarbonat oder Kaliumcarbonat. Bevorzugt wird Natriumhydroxid eingesetzt.

Als Lösemittel bei der Behandlung mit Base eignen sich Ether wie beispielsweise Diethylether, Dioxan oder Tetrahydrofuran. Besonders bevorzugt wird Tetrahydrofuran eingesetzt.

Die anschließende Cyclisierung durch Erhitzen erfolgt im allgemeinen in Lösemitteln, die sich unter den Reaktionsbedingungen nicht verändern. Hierzu gehören Kohlenwasserstoffe wie Benzol, Toluol, Xylol, Erdölfraktionen oder Tetralin oder Diglym oder Triglym. Besonders bevorzugt werden Benzol, Toluol oder Xylol eingesetst. Ebenso ist es möglich, Gemische der genannten Lösemittel einzusetzen. Besonders bevorzugt verwendet man Toluol. Das hierbei abgespaltene Wasser kann durch azeotrope Destillation oder mittels Molsieb entfernt werden. Bevorzugt wird die azeotrope Destillation.

Die Behandlung der Ester mit Basen erfolgt im allgemeinen in einem Temperaturbereich von 0°C bis 50°C, bevorzugt von 10°C bis 30°C, besonders bevorzugt bei Raumtemperatur.

Die Cyclisierung erfolgt im allgemeinen in einem Temperaturbereich von 0°C bis +200°C, bevorzugt von +25°C bis +150°C. Bevorzugt wird bei der Siedetemperatur des azeotropen Gemisches des jeweils verwendeten Lösemittels mit Wasser gearbeitet.

Die chromatographische Trennung der racemischen Lactone in die einzelnen enantiomerenreine Lactone erfolgt im allgemeinen an üblichen chiralen Materialien. Hierzu gehören bevorzugt optisch aktive Polymere von optisch aktiven (Meth)-acrylsäure-Derivaten. Besonders bevorzugt sind hier Polymerisate von optisch aktiven N-(Meth)acryloyl-aminosäure-Derivaten, wie sie in EP 379 917 beschrieben sind. Ganz besonders bevorzugt seien hier Polymerisate aus folgenden optischaktiven N-Acryloyl-aminosäureestern genannt: N-Acryloyl-L- bzw. D-aminosäurementhylester wobei als Aminosäure z.B. Leucin, Alanin, Phenylalanin, Valin oder sonstige Aminosäuren in Frage kommen.

Als Fließmittel für die Trennung des Racemats werden übliche organische Lösemittel bzw. Losemittelgemische verwendet, die das als Adsorbens eingesetzte Polymerisat anquellen und das zu benennende Racemat lösen. Beispielsweise seien genannt: Kohlenwasserstoffe wie Benzol, Toluol oder Xylol, Ether wie Diethylether, Dioxan oder Tetrahydrofuran, Halogenkohlenwasserstoffe wie Di- oder Trichlormethan, Aceton, Acetonitril oder Essigester oder aber Gemische der genannten Lösemittel. Als besonders geeignet haben sich Mischungen aus Toluol und Tetrahydrofuran sowie aus Toluol und Dioxan erwiesen.

Die Hydrolyse des jeweiligen enantiomerenreinen Lactons in das gewünschte enantiomerenreine Endprodukt erfolgt in üblicher Weise unter Verwendung von Base in organischen Lösemitteln.

Als Lösemittel kommen hierbei die üblichen organischen Lösemittel in Betracht, die sich unter den Reaktionsbedingungen nicht verändern. Bevorzugt seien hier Ether wie Diethylether, Dioxan oder Tetrahydrofuran genannt. Besonders bevorzugt wird Tetrahydrofuran eingesetzt.

Als Basen eignen sich die üblichen anorganischen Basen wie Alkalihydroxide oder Alkalicarbonate. Bevorzugt sind Natriumhydroxid oder Kaliumhydroxid.

Die Hydrolyse erfolgt im allgemeinen in einem Temperaturbereich von 0°C bis +60°C, bevorzugt von +10°C bis +50°C, besonders bevorzugt bei Raumtemperatur.

Das erfindungsgemäße Verfahren hat den Vorteil, daß die enantiomerenreinen Produkte völlig unproblematisch und vor allem ohne Verwendung weiterer Reagenzien wie beispielsweise chirale Amine aus den racemischen Estern hergestellt werden können, wobei die Gesamtausbeute des erfinderischen Verfahrens weitaus höher ist als die des aus dem Stand der Technik bekannten Verfahrens über diastereomere Amide. Darüber hinaus wird die Verunreinigung des durch Chromatographie zu trennenden Gemisches durch zusätzliche Chemikalien vermieden. Ein weiterer großer Vorteil ist, daß zur Lactonisierung die entsprechenden racemischen Ester in Form des Rohproduktes eingesetzt werden können.

### Experimenteller Teil

### Verbindung 1

### (±)-Methyl-erythro-(E)-7-[4-(4-flurophenyl)-2,6-diisopropyl-5-methoxymethyl-pyrid-3-yl]-3,5-dihydroxy-hept-6-enoat

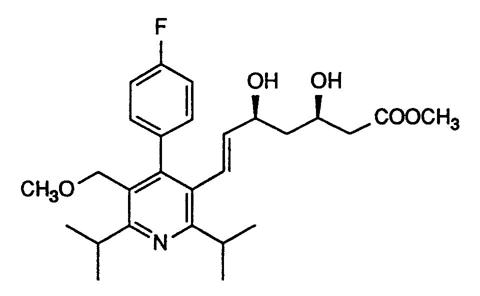

Die Herstellung erfolgt analog dem in EP 325 130 beschriebenen Verfahren. Für die folgende Lactonisierung kann jedoch das Rohprodukt eingesetzt werden.

### Lactonisierung

### Verbindung 2

### (±)-trans-(E)-6-[2-(2,6-Diisopropyl-4-(4-fluorphenyl)-3-methoxymethyl-pyrid-5-yl)-ethenyl]-3,4,5,6-tetrahydro-4-hydroxy-2H-pyran-2-on

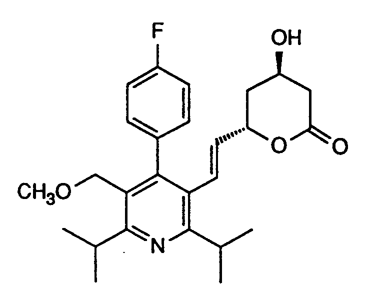

243,3 g (0,52 mol) Rohprodukt der Verbindung 1 werden in 400 ml Tetrahydrofuran p.A. gelöst und nach Zugabe von 520 ml 1 N Natronlauge rührt man 2 h bei Raumtemperatur. Das Tetrahydrofuran wird im Vakuum am Rotationsverdampfer abgezogen, der waßrige Rückstand wird mit 200 ml Wasser verdünnt und dann mit 5 N Salzsäure auf pH 4 eingestellt. Anschließend wird dreimal mit je 200 ml Methylenchlorid extrahiert, die vereinigten organischen Phasen werden mit Natriumsulfat getrocknet. Nach dem Einengen am Rotationsverdampfer wird der Rückstand in 500 ml Toluol aufgenommen und 18 h am Wasserabscheider unter Rückfluß erhitzt. Nach Abkühlen und Einengen am Rotationsverdampfer wird der feste Rückstand mit Cyclohexan verrieben und über Kieselgel gereinigt (1,5 kg, Laufmittel Essigester/Petrolether 4/6→5/5).

Ausbeute: 144 g 2(62,7 % der Theorie).
- ¹H-NMR (CDCl₃):: δ (ppm) = 1,24 (d, 6H); 1,32 (dd, 6H); 1,50 (m, 1H); 1,68 (m, 1H); 2,53 (m, 1H); 2,64 (m, 1H); 3,19 (s, 3H); 3,28 (sept., 1H); 3,37 (sept., 1H); 4,06 (s, 2H); 4,16 (m, 1H); 5,07 (m, 1H); 5,28 (dd, 1H); 6,39 (d, 1H); 7,0-7,2 (m, 4H).

### Racemattrennung

### Verbindung 3 und 4

### (+)-trans-(E)-6-[2-(2,6-Diisopropyl-4-(4-fluorphenyl)-3-methoxymethyl-pyrid-5-yl)-ethenyl]-3,4,5,6-tetrahydro-4-hydroxy-2H-pyran-2-on (3)

### (-)trans-(E)-6-[2-(2,6-Diisopropyl-4-(4-fluorphenyl)-3-methoxymethyl-pyrid-5-yl)-ethenyl]-3,4,5,6-teyahydro-4-hydroxy-2H-pyran-2-on (4)

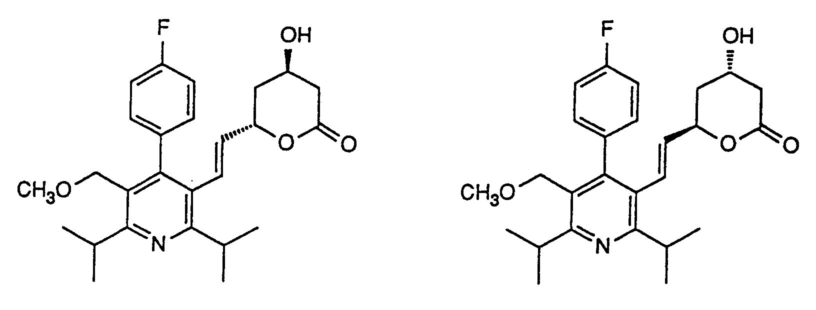

Auf eine Glassäule (⌀ 10 cm, Länge 40 cm) gefüllt mit ca. 600 g Baychiral PM (Polymerisat aus N-Acryloyl-L-phenylalanin-menthylester werden pro Lauf 6,5 g der Verbindung des Beispiels 2 aufgegeben und mit Toluol/Tetrahydrofuran 5/1 bei einem Fluß von 3,5 ml/min eluiert. Eine Trennung dauert ca. 7,5 h, so daß pro Tag 20 g getrennt werden können. Die Enantiomeren werden dabei nahezu basisliniengetrennt. Die vereinigten Produktfraktionen werden eingeengt und in einer minimalen Menge Methyl-tert.butylether aufgelöst. Durch Zugabe von n-Heptan wird 3 ausgefällt und anschließend abgesaugt und getrocknet.

Ausbeute: 3,05 g (47,5 %).

Spezifische Drehung (CDCl₃): [d]²⁰ = 35,7°C (c = 1).

Analog kann auch das (-)-Lacton 4 durch Vereinigung der entsprechenden Produktfraktionen erhalten werden.

Spezifische Drehung (EtOH): [d]²⁰ = 29,9° (c = 1).

### Hydrolyse

### Verbindung 5

### 3R,5S-(+)-Natrium-erythro-(E)-7-[4-(4-fluorphenyl)-2,6-diisopropyl-5-methoxymethyl-pyrid-3-yl]-3,5-dihydroxy-hept-6-enoat

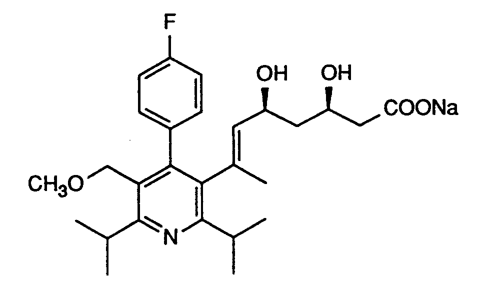

121,5 g (0,276 mol) der Verbindung 3 werden in 1000 ml absol. Tetrahydrofuran gelöst und nach Zugabe von 276 ml 1 N Natronlauge und 724 ml dest. Wasser 1,5 h bei Raumtemperatur gerührt. Das Tetrahydrofuran wird dann im Vakuum abgezogen und die verbleibende wäßrige Losung wird eingefroren und gefriergetrocknet. Das Lyophilisat wird über Phosphorpentoxid nachgetrocknet.

Ausbeute: 132,6 g (99,8 %).

Spezifische Drehung (EtOH): [d]²⁰ = 22,8° (c = 1).

## Patentansprüche

1. Verfahren zur Herstellung von 3R,5S-(+)-Natrium-erythro-(E)-7-[4-(4-fluorphenyl)-2,6-diisopropyl-5-methoxymethyl-pyrid-3-yl]-3,5-dihydroxy-hept-6-enoat der Formel 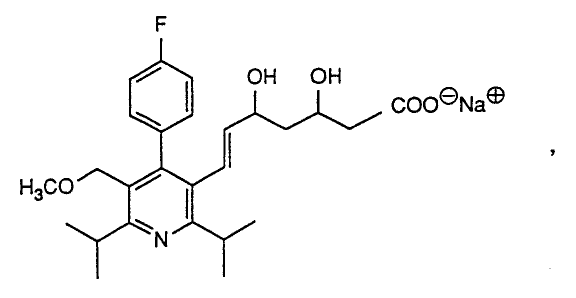 das in enantiomerenreiner Form hergestellt werden kann, indem man zunächst racemische Ester der Formel (II) 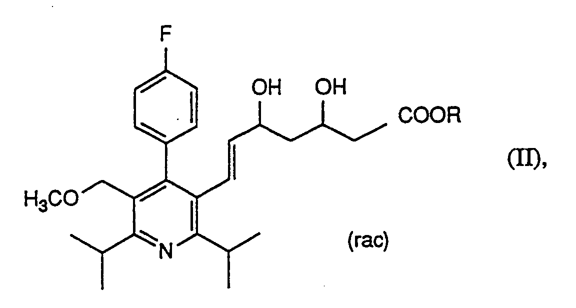 in welcher
R für C₁-C₄-Alkyl steht,
in die racemischen Lactone der Formel (III) überführt, 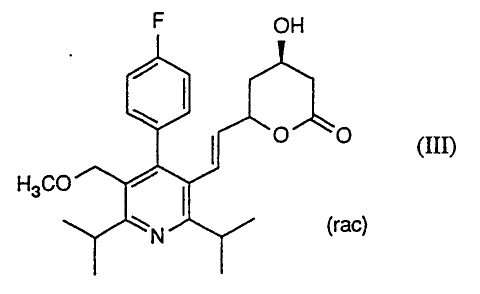 dieses Racemat durch Chromatographie an einer chiralen Phase aus optisch aktiven Polymeren von optisch aktiven (Meth)acrylsäure-Derivaten in die einzelnen enantiomeren Lactone separiert und die enantiomeren Lactone jeweils durch Hydrolyse in das gewünschte enantiomerenreine Endprodukt überführt, wobei als Fließmittel Kohlenwasserstoffe, Ether, Halogenkohlenwasserstoffe, Aceton, Acetonnitril, Essigester oder Gemische dieser Lösungsmittel eingesetzt werden.

2. Verfahren nach Anspruch 1, dadurch gekennzeichnet, daß man als Base im ersten Schritt Natriumhydroxid verwendet.

3. Verfahren nach Anspruch 1 bis 2, dadurch gekennzeichnet, daß man die Umsetzung mit Base in Tetrahydrofuran durchführt.

4. Verfahren nach Anspruch 1 bis 3, dadurch gekennzeichnet, daß man die Cyclisierung in Toluol durchführt.

5. Verfahren nach Anspruch 1 bis 4, dadurch gekennzeichnet, daß man die Cyclisierung in einem Temperaturbereich von 0°C bis +200°C durchführt.

6. Verfahren nach Anspruch 1 bis 5, dadurch gekennzeichnet, daß als chirale Phase für die chromatographische Trennung ein Perlpolymerisat aus N-Acryloyl-aminosäureestern verwendet.

7. Verfahren nach Anspruch 1 bis 6, dadurch gekennzeichnet daß man als chirale Phase ein Perlpolymerisat aus N-Acryloyl-L-phenylalanin-menthyl ester verwendet.

8. Verfahren nach Anspruch 1 bis 8, dadurch gekennzeichnet, daß man als Fließmittel für die Trennung des Racemats Gemische Von Toluol und Dioxan bzw. von Toluol und Tetrahydrofuran einsetzt.

9. (+)-Trans-(E)-6-[2-(2,6-diisopropyl-4-(4-fluorphenyl)-3-methoxymethylpyrid-5-yl)-ethenyl]-3,4,5,6-tetrahydro-4-hydroxy-2H-pyran-on der Formel

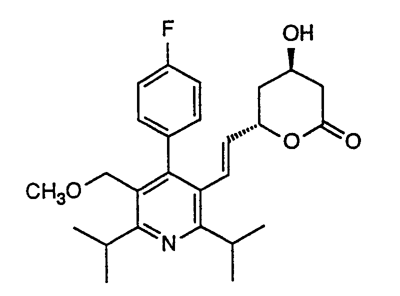

## Claims

1. Process for preparing sodium 3R,5S-(+)-erythro-(E)-7-[4-(4-fluorophenyl)-2,6-diisopropyl-5-methoxymethyl-pyrid-3-yl]-3,5-dihydroxy-hept-6-enoate of the formula 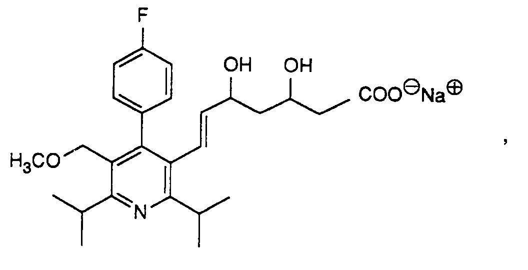 which can be prepared in enantiomerically pure form by initially converting racemic esters of the formula (II) 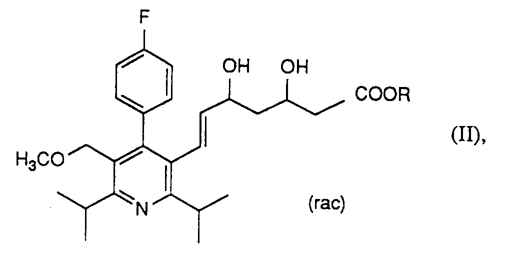 in which
R represents C₁-C₄-alkyl,
into the racemic lactones of the formula (III), 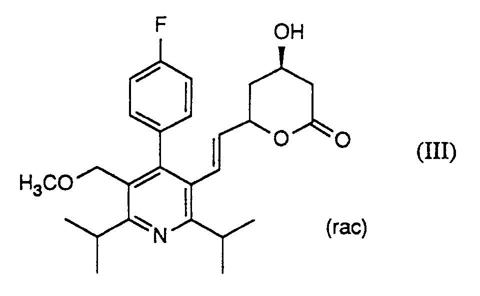 separating this racemate into the individual enantiomeric lactones by chromatography on a chiral phase consisting of optically active polymers of optically active (meth)acrylic acid derivatives, and converting the enantiomeric lactones, in each case by hydrolysis, into the desired enantiomerically pure end product, hydrocarbons, ethers, halohydrocarbons, acetone, acetonitrile, ethyl acetate or mixtures of these solvents being used as the mobile solvent.

2. Process according to Claim 1, characterized in that sodium hydroxide is used as the base in the first step.

3. Process according to Claim 1 or 2, characterized in that the reaction with the base is carried out in tetrahydrofuran.

4. Process according to Claims 1 to 3, characterized in that the cyclization is carried out in toluene.

5. Process according to Claims 1 to 4, characterized in that the cyclization is carried out in a temperature range from 0°C to +200°C.

6. Process according to Claims 1 to 5, characterized in that a bead polymer consisting of N-acryloylamino acid esters is used as the chiral phase for the chromatographic separation.

7. Process according to Claims 1 to 6, characterized in that a bead polymer consisting of N-acryloyl-L-phenylalanine menthyl ester is used as the chiral phase.

8. Process according to Claims 1 to 8, characterized in that mixtures of toluene and dioxane, or of toluene and tetrahydrofuran, are employed as the mobile solvent for separating the racemate.

9. (+)-Trans-(E)-[2-(2,6-diisopropyl-4-(4-fluorophenyl)-3-methoxymethyl-pyrid-5-yl)-ethenyl]-3,4,5,6-tetrahydro-4-hydroxy-2H-pyran-2-one of the formula

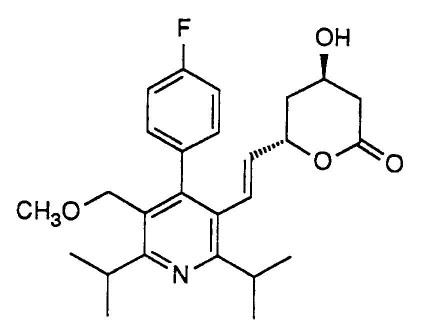

## Revendications

1. Procédé de production du 3R,5S-(+)-érythro-(E)-7-[4-(4-fluorophényl)-2,6-diisopropyl-5-méthoxyméthyl-pyrid-3-yl]-3,5-dihydroxy-hept-6-énoate de sodium de formule 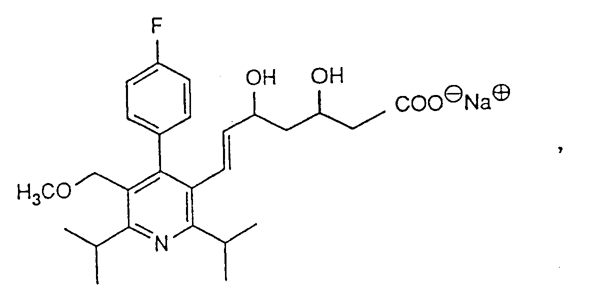 qui peut être préparé sous la forme énantiomère pur, par le fait qu'on transforme tout d'abord des esters racémiques de formule (II) 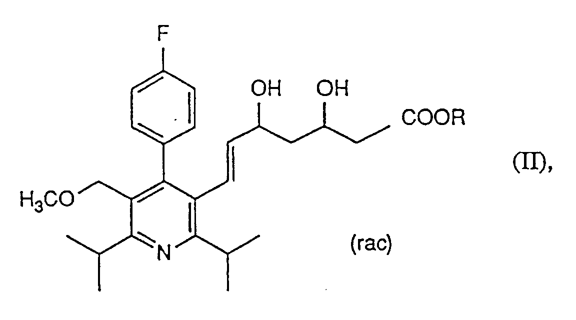 dans laquelle
R est un groupe alkyle an C₁ à C₄,
en les lactones racémiques de formule (III) 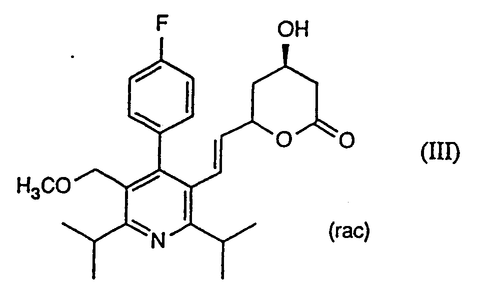 sur une phase chirale formée de polymères optiquement actifs de dérivés d'acide (méth)acrylique optiquement actifs, on fractionne ce racémate par chromatographie en les lactones énantiomères individuelles et on transforme chacune des lactones énantiomères par hydrolyse en le produit final énantiomère pur souhaité, en utilisant comme éluants des hydrocarbures, des éthers, des hydrocarbures halogénés, des acétones, l'acétonitrile, l'acétate d'éthyle ou des mélanges de ces solvants.

2. Procédé suivant la revendication 1, caractérisé en ce qu'on utilise l'hydroxyde de sodium comme base dans la première étape.

3. Procédé suivant les revendications 1 et 2, caractérisé en ce qu'on conduit la réaction avec la base dans du tétrahydrofuranne.

4. Procédé suivant les revendications 1 à 3, caractérisé en ce qu'on conduit la cyclisation dans du toluène.

5. Procédé suivant les revendications 1 à 4, caractérisé en ce qu'on conduit la cyclisation dans une plage de températures de 0°C à +200°C.

6. Procédé suivant les revendications 1 à 5, caractérisé en ce qu'on utilise un produit de polymérisation en suspension d'esters de N-acryloylaminoacide comme phase chirale pour la séparation chromatographique.

7. Procédé suivant les revendications 1 à 6, caractérisé en ce qu'on utilise comme phase chirale un produit de polymérisation en suspension obtenu à partir d'ester de menthyle de N-acryloyl-L-phénylalanine.

8. Procédé suivant les revendications 1 à 8, caractérisé en ce qu'on utilise comme éluant pour le fractionnement du racémate des mélanges de toluène et de dioxanne ou de toluène et de tétrahydrofuranne.

9. La (+)-trans-(E)-6-[2-(2,6-diisopropyl-4-(4-fluorophényl)-3-méthoxyméthyl-pyrid-5-yl)-éthényl]-3,4,5,6-tétrahydro-4-hydroxy-2H-pyranne-2-one de formule
